# EUROPEAN PATENT SPECIFICATION

(11) **EP 3 834 871 B1**
(45) Date of publication and mention of the grant of the patent: **16.04.2025**
(21) Application number: 18931204.4
(22) Date of filing: 21.08.2018
(51) Int. Cl.: A61M 16/00, A61B 5/087, A61B 5/00, G16H 20/40, G16H 40/67, G16H 50/20

(54) **COMPUTER PROGRAM FOR VENTILATION TRIGGER DETECTION, VENTILATION TRIGGER DETECTION APPARATUS, AND VENTILATION DEVICE**
COMPUTERPROGRAMM ZUR ERKENNUNG DES VENTILATORAUSLÖSERS, VORRICHTUNG ZUR ERKENNUNG DES VENTILATORAUSLÖSERS UND VENTILATOR
PROGRAMME INFORMATIQUE POUR LA DÉTECTION DE DÉCLENCHEMENT DE VENTILATION, APPAREIL DE DÉTECTION DE DÉCLENCHEMENT DE VENTILATION ET DISPOSITIF DE VENTILATION

(43) Date of publication of application: 16.06.2021
(73) Proprietor: Shenzhen Mindray Bio-Medical Electronics Co., Ltd., Shenzhen, Guangdong 518057 (CN); Peking Union Medical College Hospital, Chinese Academy of Medical Science and Peking Union Medical College, Beijing 100730 (CN)
(72) Inventor: XU, Jun, Beijing 100730 (CN); LIU, Jinglei, Shenzhen, Guangdong 518057 (CN); YU, Xuezhong, Beijing 100730 (CN); FU, Yangyang, Beijing 100730 (CN); ZOU, Xinru, Shenzhen, Guangdong 518057 (CN); ZHOU, Xiaoyong, Shenzhen, Guangdong 518057 (CN)
(74) Representative: KIPA AB
(86) International application number: PCT/CN2018/101606
(87) International publication number: WO 2020/037519

(56) References cited:
- CN-A- 102 266 614
- CN-A- 103 920 215
- CN-A- 106 840 734
- CN-A- 106 840 734
- KR-A- 20110 094 649
- US-B2- 8 757 153
- US-B2- 9 392 964
- BEHNOOD GHOLAMI ET AL: "Replicating human expertise of mechanical ventilation waveform analysis in detecting patient-ventilator cycling asynchrony using machine learning", COMPUTERS IN BIOLOGY AND MEDICINE, vol. 97, 1 June 2018 (2018-06-01), US, pages 137 - 144, XP055608318, ISSN: 0010-4825, DOI: 10.1016/j.compbiomed.2018.04.016

## Description

### TECHNICAL FIELD

The disclosure relates to the field of medical technologies, and in particular to a ventilation trigger detection method and apparatus, a ventilation device and a storage medium.

### BACKGROUND

Ventilators, as an effective means capable of artificially replacing a function of spontaneous ventilation, have been widely used in respiratory failure, anesthesia and respiration management, respiratory support therapy and emergency resuscitation caused by various causes. During mechanical ventilation for users by using the ventilators, patient-ventilator incoordination or patient-ventilator out-of-synchronization caused by abnormal ventilation (such as ineffective effort) often occurs, and the ventilators in the related art may not identify abnormal events or patient-ventilator out-of-synchronization during the ventilation.

US9392964 discloses a controller or processor for implementing detection of asynchrony, where automated determination of feature sets is implemented to design different asynchrony event classifiers, and the feature sets are compared with a set of stored data thresholds.

US8757153 discloses detecting the double triggering detection. The method include analyzing the processed ventilatory parameter data, which includes receiving one or more predetermined thresholds associated with the processed ventilatory parameter data and detecting whether the processed ventilatory parameter data breaches the one or more predetermined thresholds.

Gholami (*"Replicating human expertise of mechanical ventilation waveform analysis in detecting patient-ventilator cycling asynchrony using machine learning ", Computers in Biology and Medicine*) discloses automatic detection of asynchrony event using the machine learning method for cycling asynchrony.

### SUMMARY

In view of this, this disclosure provides a ventilation trigger detection method and apparatus, a ventilation device, which are capable of accurately determining the patient-ventilator synchrony during mechanical ventilation.

The present invention is defined by the appended claims only, Reference(s) to "embodiments" throughout the description which are not under the scope of the appended claims merely represents possible exemplary executions and are therefore not part of the present invention.

### BRIEF DESCRIPTION OF THE DRAWINGS

FIG. 1A is a first schematic diagram of a composition structure of a ventilation trigger detection apparatus provided by an embodiment of the disclosure;
FIG. 1B is a schematic diagram of a composition structure of a ventilation device provided by an embodiment of the disclosure;
FIG. 2 is a schematic diagram of a waveform of one respiratory cycle of a ventilator in a pressure trigger mode provided by an embodiment of the disclosure;
FIG. 3 is a schematic flowchart of a ventilation trigger detection method provided by an example of the disclosure;
FIG. 4 is a schematic diagram of waveforms of pressure trigger and flow trigger in a pressure support mode provided by an embodiment of the disclosure;
FIG. 5 is a schematic diagram of waveforms of normal inspiration-to-expiration switching in the pressure support mode provided by an embodiment of the disclosure;
FIG. 6 is a schematic diagram of a flow-time waveform of ineffective effort in the pressure support mode;
FIG. 7 is a schematic diagram of waveforms of ineffective effort in the pressure support mode provided by an embodiment of the disclosure;
FIG. 8 is a schematic diagram of waveforms of double trigger provided by an embodiment of the disclosure;
FIG. 9 is a schematic diagram of waveforms of false trigger provided by an embodiment of the disclosure;
FIG. 10 is a schematic diagram of waveforms of delayed cycling provided by an embodiment of the disclosure;
FIG. 11 is a schematic diagram of waveforms of premature cycling provided by an embodiment of the disclosure; and
FIG. 12 is a second schematic diagram of a composition structure of the ventilation trigger detection apparatus provided by the embodiment of the disclosure.

### DESCRIPTION OF THE PREFERRED EMBODIMENTS

The disclosure will be further described below in detail in conjunction with the accompanying drawings and the embodiments. It should be understood that the embodiments provided herein are merely intended to explain the disclosure, and are not intended to limit the disclosure. In addition, the embodiments provided below are used to implement some embodiments of the disclosure, but not all embodiments for implementing the disclosure. In the case of no conflict, the technical solutions recorded in the embodiments of the disclosure may be implemented in any combination.

It should be noted that the term "first/second/third" in the embodiments of the disclosure is only used to distinguish similar objects, and does not represent specific order for the objects. It may be understood that "first/second/third" may be interchanged for specific order or chronological order when allowed. It should be understood that the objects distinguished by "first/second/third" may be interchangeable where appropriate, so that the embodiments of the disclosure described herein can be implemented in an order other than that illustrated or described herein.

The inventors have found in the course of their studies that during mechanical ventilation, a patient needs, when spontaneously respiring, to make an inspiratory or expiratory effort to reach a set inspiratory trigger (which may be set by setting a pressure or flow trigger sensitivity)/expiratory switching (which may be set according to a percentage of an inspiratory flow peak) sensitivity so that a ventilator can be switched to a corresponding inspiratory or expiratory phase. For example, the inspiratory trigger may be set as flow trigger, the inspiratory phase may be enabled when a flow exceeds a trigger sensitivity (e.g., 2 L/min), or in a pressure trigger mode, the inspiratory phase may be enabled when an airway pressure is below a positive end expiratory pressure (PEEP)-trigger sensitivity (e.g., 2 cmH2O). The expiratory trigger sensitivity may be generally a percentage of an inspiratory peak flow, for example, the ventilator may be switched to the expiratory phase when the inspiratory flow decreases to 25% of the inspiratory peak flow. Since the inspiratory or expiratory trigger sensitivity is set by a doctor based on experience, the situation clinically may occur that the trigger sensitivity setting of the ventilator may be inconsistent with the demand of a patient, resulting in the occurrence of patient-ventilator out-of-synchronization events, such as ineffective effort, double trigger, delayed cycling, premature cycling and delayed inspiratory, and then the use effect of the user may be influenced.

Before describing the embodiments of the disclosure in further detail, the nouns and terms involved in the embodiments of the disclosure are explained, and the nouns and terms involved in the embodiments of the disclosure are applicable to the following explanation.
(1) Flow trigger may refer to that a continuous air flow is delivered in a ventilator loop, and a ventilator detects air flow velocities at an inlet end and an outlet end of a breathing circuit, and is triggered to deliver gas when a difference between the air flow velocities at the two ends reaches a preset level.
(2) Pressure trigger may refer to that a pressure in an airway drops when a user inhales, the ventilator detects the pressure change and starts gas delivery, so that synchronous inhalation is completed.
(3) Tidal volume may refer to the volume of gas inhaled or exhaled each time when the user breathes quietly. It is related to the age, gender, volume surface, breathing habits and body metabolism of the user. The tidal volume set by the ventilator is usually referred to as an inspired gas volume and may be adjusted according to the blood gas analysis of the user.
(4) Patient-ventilator out-of-synchronization may refer to that a respiratory cycle of a ventilation device, such as a ventilator, is not coordinated with a patient.

A ventilation trigger detection apparatus provided by an embodiment of the disclosure will be described below. The ventilation trigger detection apparatus provided by the embodiment of the disclosure may be implemented in hardware, software or a combination of hardware and software, and various exemplary implementations of the ventilation trigger detection apparatus provided in the embodiment of the disclosure will be described below.

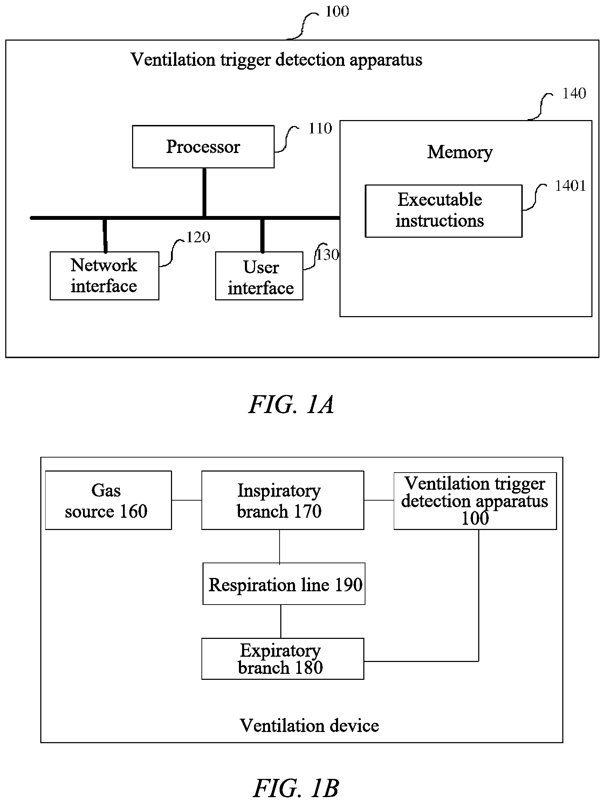

A hardware structure of the ventilation trigger detection apparatus of the embodiment of the disclosure will be described in detail below. FIG. 1A is a schematic diagram of a composition structure of the ventilation trigger detection apparatus provided by the embodiment of the disclosure, and it will be understood that FIG. 1A only shows an exemplary structure, rather than all the structures, of the ventilation trigger detection apparatus, and that part or all of the structure shown in FIG. 1A may be implemented according to requirements. The ventilation trigger detection apparatus 100 provided by the embodiment of the disclosure may include: at least one processor 110, a memory 140, and a user interface 130. The ventilation trigger detection apparatus 100 may be further provided with a network interface 120 according to actual requirements.

The user interface 130 may include a display, a keyboard, a mouse, a trackball, a click wheel, keys, buttons, a touch pad, or a touch screen, etc.

The memory 140 may be a volatile memory or a non-volatile memory, or may include both a volatile memory and a non-volatile memory. The nonvolatile memory may be a read only memory (ROM), a programmable read-only memory (PROM), an erasable programmable read-only memory (EPROM), a flash memory, etc. The volatile memory may be a random access memory (RAM), which acts as an external cache. By way of example, and not limitation, many forms of RAMs are available, such as a static random access memory (SRAM), and a synchronous static random access memory (SSRAM). The memory 140 described in the embodiment of the disclosure is intended to include these memories and any other suitable types of memories.

The processor 110 may be an integrated circuit chip having a signal processing capability, such as a general-purpose processor, a digital signal processor (DSP), or other programmable logic devices, discrete gates or transistor logic devices and discrete hardware components, wherein the general-purpose processor may be a microprocessor or any conventional processor.

The memory 140 is capable of storing executable instructions 1401 to support operations of the ventilation trigger detection apparatus 100. Examples of these executable instructions may include: various forms of software modules, such as programs, plug-ins and scripts, configured to operate on the ventilation trigger detection apparatus 100. The programs, for example, may include an operating system and application programs, wherein the operating system contains various system programs, such as a framework layer, a core library layer and a driver layer, which are configured to implement various basic services and to process hardware-based tasks.

As an example, implemented by combining software and hardware, of the ventilation trigger detection apparatus provided by the embodiment of the disclosure, the ventilation trigger detection apparatus provided by the embodiment of the disclosure may be directly embodied as different forms of software modules executed by the processor 110, the software modules may be located in a storage medium, the storage medium may be located in the memory 140, and the processor 110 reads executable instructions included in the software modules in the memory 140 and implements a ventilation trigger detection method provided by the examples of the disclosure in combination with necessary hardware (for example, including the processor 110 and other components connected to a bus).

A ventilation device provided by the embodiment of the disclosure will be described below. FIG. 1B is a schematic diagram of a composition structure of the ventilation device provided by the embodiment of the disclosure. Referring to FIG. 1B, the ventilation device provided by the embodiment of the disclosure includes a ventilation trigger detection apparatus 100 provided by the embodiment of the disclosure, and a gas source 160, an inspiratory branch 170, an expiratory branch 180 and a respiration line 190; wherein the gas source may supply gas during mechanical ventilation; the inspiratory branch may be connected to the gas source to provide an inspiration path during the mechanical ventilation; the expiratory branch may provide an expiration path during the mechanical ventilation; the respiration line may be connected to the inspiratory branch and the expiratory branch respectively, and used for delivering gas to a user or exhausting gas from a user during the mechanical ventilation; and the ventilation trigger detection apparatus may be connected to the inspiratory branch and the expiratory branch respectively. The ventilation device provided by the embodiment of the disclosure may be a ventilator, an anesthesia machine or other devices.

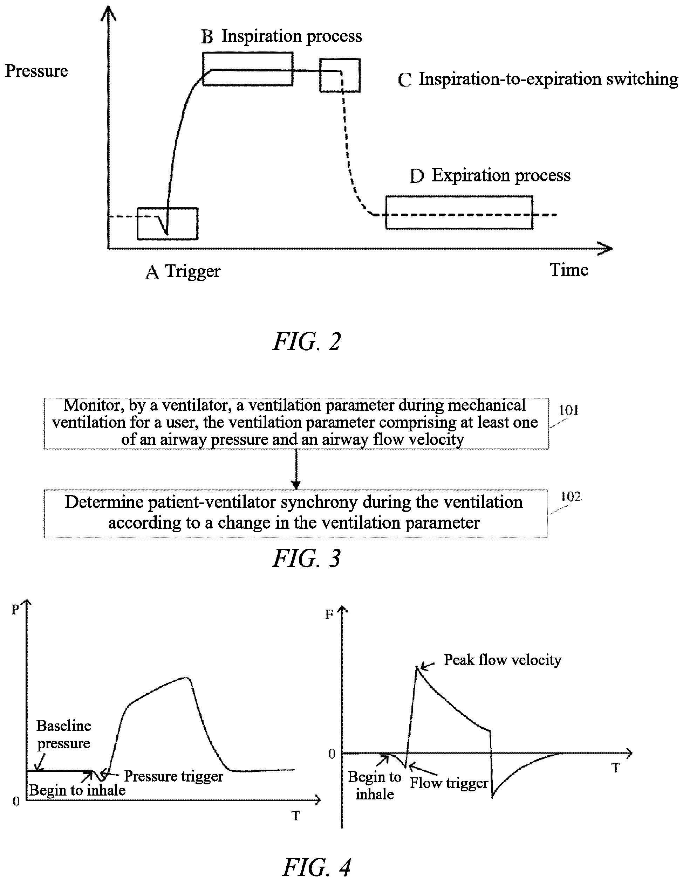

In the following, the mechanical ventilation is illustrated by taking a ventilator as an example of the ventilation device. FIG. 2 is a schematic diagram of a waveform of one respiratory cycle of the ventilator in a pressure trigger mode provided by the embodiment of the disclosure. Referring to FIG. 2, one respiratory cycle of mechanical ventilation of the ventilator may include inspiratory trigger (starting the gas delivery of the ventilator), inspiration process (gas delivery process of the ventilator), inspiration-to-expiration switching (ending the gas delivery of the ventilator) and expiration process.

In this case, a patient actively inhales, causing a pressure drop or flow change in an airway, and the ventilator may sense the inhalation action of the user and give the user one delivery of gas, which is called a user trigger. The sensing action of the ventilator may be set manually and may be controlled by adjusting the trigger sensitivity. Trigger modes of the ventilator may include, but are not limited to, flow trigger and pressure trigger.

In the inspiration process, the ventilator may output gas at a certain flow, and a certain volume and a certain pressure are generated as the gas enters a breathing circuit and the user's lungs. The inspiration-to-expiration switching of the ventilator may be controlled in the following three modes:
controlling ventilation (volume/pressure), namely enabling the ventilator to provide a constant ventilation tidal volume or pressure to the patient for ventilation, and performing time-based switching, namely performing switching when the gas delivery time reaches a set inspiratory time; and
pressure support, namely the user obtains a certain level of pressure support after triggering the ventilator to deliver the gas, and performing flow-based switching, namely performing switching when the flow drops to a percentage of a peak flow.

Continuing to take the ventilator as the example of the ventilation device, the ventilation trigger detection method provided by the example of the disclosure will be described. Referring to FIG. 3, FIG. 3 is an optional flowchart of the ventilation trigger detection method provided by the example of the disclosure. The ventilation trigger detection method of the example of the disclosure will be described with reference to the steps shown in FIG. 3.

Step 101, monitoring, by a ventilator, a ventilation parameter during mechanical ventilation for a user, the ventilation parameter may include at least one of an airway pressure and an airway flow; and
Step 102, determining patient-ventilator synchrony during the ventilation according to a change in the ventilation parameter.

The ventilator monitors an airway pressure and an airway flow simultaneously so as to learn changes in the airway pressure and the airway flow over time, the ventilator acquires waveforms of the changes in the airway pressure and the airway flow over time and learns the changes in the airway pressure and the airway flow over time by analyzing the waveforms.

During actual implementation, the ventilator detects a change in the ventilation parameter, and determines that ventilation trigger is abnormal, that is, patient-ventilator out-of-synchronization, when the change in the ventilation parameter meets a corresponding parameter change condition. Specifically, it is determined in the following way that the change in the ventilation parameter meets the corresponding parameter change condition: acquiring a waveform of a ventilation parameter over time; carrying out similarity matching on the acquired waveform and a stored waveform of the corresponding parameter; and determining that the change in the ventilation parameter meets the corresponding parameter change condition when the similarity obtained by the matching reaches a waveform similarity threshold.

During actual implementation, abnormal ventilation of the ventilator during the mechanical ventilation may include, but is not limited to an abnormal trigger event and an abnormal inspiration-to-expiration switching event. The ventilator may detect the abnormal trigger event and the abnormal inspiration-to-expiration switching event, wherein the types of abnormal trigger may include, but are not limited to: ineffective effort, double trigger and false trigger, and the types of abnormal inspiration-to-expiration switching may include, but are not limited to: delayed cycling and premature cycling. After determining the type of abnormal ventilation that has occurred on the ventilator, the ventilator may adjust the trigger sensitivity of the ventilator correspondingly according to the determined type of abnormal ventilation.

Before the patient-ventilator out-of-synchronization during the mechanical ventilation of the ventilator is described, normal trigger of gas delivery and normal inspiration-to-expiration switching of the ventilator are first described.

FIG. 4 is a schematic diagram of waveforms of pressure trigger and flow trigger in a pressure support mode provided by an embodiment of the disclosure. Referring to FIG. 4, the airway pressure may be reduced when the user inhales, and the ventilator may be triggered to enable an inspiratory phase and to start gas delivery when the airway pressure reaches a pressure trigger sensitivity; and the air flow velocities at the inlet end and the outlet end in the breathing circuit have an air flow difference when the user inhales, and the ventilator may be triggered to enable the inspiratory phase and to start gas delivery when the flow difference reaches a preset trigger sensitivity.

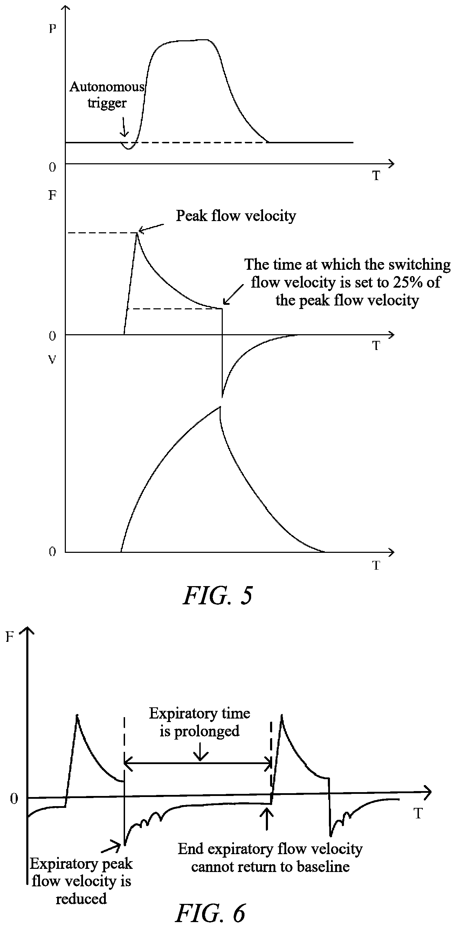

In volume control and pressure control modes, time-based switching may be performed by setting inspiratory time or an inspiration/expiration ratio, a respiratory rate, etc. Under the pressure support, a flow-based switching mode may be adopted, for example, reducing the flow to 25% of a peak flow serves as an index of flow-based switching. FIG. 5 is a schematic diagram of waveforms of normal inspiration-to-expiration switching in the pressure support mode provided by an embodiment of the disclosure. Referring to FIG. 5, the ventilator may perform inspiration-to-expiration switching when detecting that the flow reaches 25% of the peak flow.

In the following, the abnormal trigger event and the abnormal inspiration-to-expiration switching event in the patient-ventilator out-of-synchronization are explained respectively.

With regard to ineffective effort, the situation that the user has made an inhalation effort but cannot trigger the ventilator to effectively delivery gas is called ineffective effort. The ineffective effort may lead to patient-ventilator incoordination, so that inhalation work is increased, but the ventilator cannot be effectively triggered to delivery the gas, that is, trigger failure. Reasons for the ineffective efforts may include, but are not limited to, the following situations:
(1) decreased respiratory center drive: it may occur in sedation, hyperventilation, deep sleep and so on, the respiratory center drive of such populations decreases, inspiratory actions slow down, the trigger time is prolonged, and the occurrence rate of the ineffective effort increases;
(2) respiratory muscle weakness: in some disease states, the user suffers from respiratory muscle weakness, so that the inspiratory volume is insufficient to cause a pressure change in the line or cause the change in the flow to reach a trigger point, leading to ineffective effort, for example, it occurs when myasthenia gravis, Guillain-Barre syndrome and so on affect the respiratory muscle;
(3) too high trigger setting: when the trigger setting is too high, the work required to reach the trigger point increases, often leading to trigger difficulty.
(4) PEEPi: when the PEEPi occurs in the user, an end expiratory alveolar pressure increases, and the patient needs to strive to inhale to enable the alveolar pressure to reach a zero point and then drop to the trigger point such that the ventilator can be triggered to deliver the gas, increasing the work of the respiratory muscle and making trigger difficult. It is common in patients suffering from chronic obstructive pulmonary disease (COPD) and tachypnea, mostly caused by prolonged expiratory time and insufficient expiratory time due to increased expiratory resistance. The waveform is characterized by returning to a baseline by a flow-time curve method. Reference is made to FIG. 6, which is a schematic diagram of a flow-time waveform of the ineffective effort occurring in a patient with PEEPi in the pressure support mode.

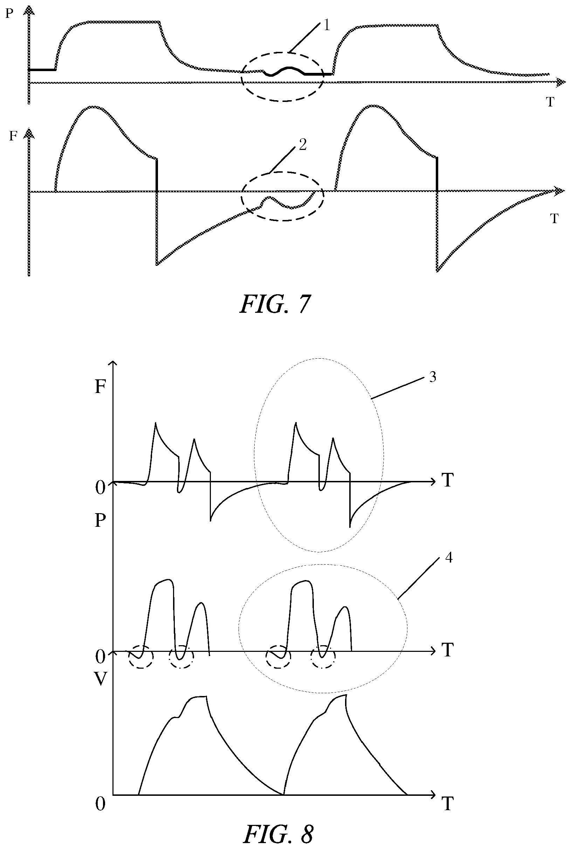

In the case of the ineffective effort described above, there are similar manifestations in the waveform of the airway pressure over time and the waveform of the airway flow over time. FIG. 7 is a schematic diagram of waveforms of ineffective effort in the pressure support mode provided by an embodiment of the disclosure. Referring to FIG. 7, parts indicated by reference numbers 1 and 2 show waveforms of the ineffective effort, which represent that the user performs an inhalation operation, but since the trigger sensitivity is not reached, the part indicated by the reference number 1 shows the situation that the pressure drops and then rises, the part indicated by the reference number 2 shows the situation that the slope of the flow suddenly increases (exceeds a preset threshold but does not reach the trigger sensitivity) and then becomes smaller, and the time corresponding to a turning point of the pressure change indicated by the reference number 1 and the moment of sudden change in the slope of the flow indicated by the reference number 2 may be the corresponding time of occurrence of an ineffective effort event.

When the ventilator detects that at least one of the following situations has occurred by analyzing waveforms of the acquired ventilation parameters (the airway pressure and the airway flow) over time, it may be determined that an abnormal event of ineffective effort has occurred on the ventilator, that is, it may be determined that the change in the ventilation parameters meets corresponding parameter change conditions:
the change trend of the airway pressure over time at the expiratory stage may be drop and then rise, and a minimum value of the airway pressure in a first time period may be greater than the PEEP-pressure trigger sensitivity of the ventilator; or
the change rate of the airway flow over time at the expiratory stage appears at a time point when the change rate exceeds a change rate threshold, and the airway flow corresponding to the time point is smaller than the flow trigger sensitivity of the ventilator.

Accordingly, in the above situations, the time corresponding to the turning point at which the pressure at the expiratory stage drops and then rises, or the time at which the change rate of the airway flow over time at the expiratory stage exceeds the change rate threshold may be the time of occurrence of an ineffective effort.

In an embodiment, the ventilator may determine the occurrence of the ineffective effort when detecting that a valley appears in an airway pressure-time waveform and/or an accelerated rise appears in a ventilation flow-time waveform at an expiratory stage, and inspiratory trigger of the ventilator is not enabled.

In an embodiment, a pressure-time waveform graph and a flow-time waveform graph corresponding to the ineffective effort event may be stored in the ventilator, a corresponding waveform graph may be obtained by monitoring the airway pressure and/or airway flow during the mechanical ventilation by the ventilator, and the obtained waveform graph may be matched with the stored corresponding waveform graph of ineffective effort. For example, the obtained pressure-time waveform graph may be matched with the stored pressure-time waveform graph corresponding to the ineffective effort, and when the similarity obtained by matching reaches a similarity threshold (e.g., 0.9), it may be determined that an abnormal event of ineffective effort has occurred on the ventilator, and thus abnormality prompt information indicating that ineffective effort has occurred on the ventilator may be sent.

In an embodiment, the ventilator may be triggered to enable the inspiratory phase, that is, the ventilator may be triggered to start gas delivery when determining that the abnormal event of ineffective effort has occurred on the ventilator.

In an embodiment, the ventilator may send abnormality prompt information, by means of a user interface (UI) and/or in the form of sound, indicating that the ineffective effort has occurred on the ventilator when determining that the abnormal event of ineffective effort has occurred, so that the user may adjust the trigger sensitivity to better realize the patient-ventilator synchronization.

A preset trigger sensitivity adjusting strategy is adopted to adjust the trigger sensitivity of the ventilator when the ventilator determines that the abnormal event of ineffective effort has occurred, for example, the trigger sensitivity is periodically reduced (i.e. in each subsequent respiratory cycle, the trigger sensitivity is reduced to 90% of that in a previous cycle each time) until no ineffective effort event occurs. Alternatively, trigger determination may be carried out by determining the change trend of the flow or pressure waveform, if the slope of the flow gradually increases to a certain threshold, it may be considered that an inhalation effort has made to trigger the ventilator to delivery gas.

With regard to double trigger, due to improper setting of the trigger sensitivity of the ventilator, the ventilator may be repeatedly triggered within a short time (e.g., 1 s), causing patient-ventilator incoordination, the patient has breathing difficulty and cannot reach a preset tidal volume or minute ventilation volume of the ventilator, and the ventilation quality may be reduced. Reasons for the double trigger may include, but are not limited to, the following situations:
(1) too low inspiratory flow: the inhaling action will be made again when the flow of the ventilator is set improper to make the patient feel that the delivery flow cannot meet the demand of the body, so that the inspiratory trigger sensitivity is achieved, and the ventilator is triggered again to delivery gas;
(2) too low tidal volume: the patient will have to inhale again when the tidal volume of the ventilator is set too low to meet the demand of the patient, and after the trigger sensitivity is reached, the ventilator is triggered to delivery gas;
(3) improper setting of expiration switching: the inhalation effort is again initiated, resulting in repeated trigger of the ventilator, when the ventilator performs premature switching to cause the patient to inhale not enough gas to meet his/her own need.

In the case of the double trigger described above, there are similar manifestations in the waveform of the airway pressure over time and the waveform of the airway flow over time. FIG. 8 is a schematic diagram of waveforms of double trigger provided by an embodiment of the disclosure. Referring to FIG. 8, the ventilator may be triggered twice to enable the inspiratory phase in one respiratory cycle, as shown by parts indicated by the reference numbers 3-4 in FIG. 8.

In an embodiment, when the ventilator detects that at least one of the following situations has occurred by analyzing waveforms of the acquired ventilation parameters (the airway pressure and the airway flow) over time, it may be determined that an abnormal event of double trigger has occurred on the ventilator, that is, it may be determined that the change in the ventilation parameters meets corresponding parameter change conditions:
the airway pressure may reach the pressure trigger sensitivity at least twice in a third time period; and
the airway flow may reach the flow trigger sensitivity at least twice in the third time period.

In practical application, the third time period described herein may be set according to actual requirements, for example, the third time period may be set to correspond to an exhalation time constant of the patient.

In an embodiment, the ventilator may determine the occurrence of the double trigger when detecting that two sections of inspiratory pressure waveforms appear in the airway pressure-time waveform at an inspiratory stage and/or a short-time expiratory cycle appears between two inspiratory cycles in the airway flow-time waveform. The user's expiratory stage herein may be defined by a waveform, and the part in the waveform other than the expiratory stage is the inspiratory stage; and the term short-time refers to a time smaller than a preset time threshold, and the time threshold value may be set according to actual demands, such as twice the time constant, wherein the time constant is equal to the product of an airway resistance and a compliance.

In an embodiment, a pressure-time waveform graph and a flow-time waveform graph corresponding to the double trigger event may be stored in the ventilator, a corresponding waveform graph may be obtained by monitoring the airway pressure and/or airway flow during the mechanical ventilation by the ventilator, and the obtained waveform graph may be matched with the stored corresponding waveform graph of double trigger. For example, the obtained pressure-time waveform graph may be matched with the stored pressure-time waveform graph (within one respiratory cycle) corresponding to the double trigger, and when the similarity obtained by matching reaches a similarity threshold (e.g., 0.9), it may be determined that an abnormal event of double trigger has occurred on the ventilator, and thus abnormality prompt information indicating that double trigger has occurred on the ventilator is sent.

In an embodiment, the ventilator may send the abnormality prompt information, by means of a UI and/or in the form of sound, indicating that double trigger has occurred on the ventilator when determining that the abnormal event of double trigger has occurred, so that the user adjusts ventilation (e.g., the tidal volume, the inspiratory pressure or inspiratory time) to better realize the patient-ventilator synchronization.

A preset ventilation adjusting strategy is adopted to adjust the tidal volume/inspiratory pressure/inspiratory time of ventilation and the inspiratory trigger sensitivity when the ventilator determines that the abnormal event of double trigger has occurred, the trigger sensitivity is periodically reduced (i.e., in each subsequent respiratory cycle, the trigger sensitivity is reduced to 90% of that in a previous cycle each time) until no double trigger event occurs.

With regard to false trigger, the patient does not make the inspiratory effort, and due to the fact that the trigger sensitivity of the ventilator is set too low, a pipeline leaks or accumulated water of the line shocks, the pressure in the line changes to trigger the ventilator to delivery gas, which is called self-trigger of the ventilator and also called false trigger. Reasons for the false trigger may include, but are not limited to, the following situations:
(1) too lower trigger setting: a pressure change in a ventilator circuit often occurs due to the shake of the line and the shock of accumulated water in the line, causing false trigger, when the trigger sensitivity of the ventilator is set too low;
(2) water accumulation of the line: if the trigger sensitivity of the ventilator is properly set while a large amount of water is accumulated in the line, the pressure in the ventilator circuit may be suddenly reduced to cause self-trigger of the ventilator when the accumulated water is poured into an accumulated water bottle by the shaking the line;
(3) line gas leakage: gas delivery of the ventilator is induced when leakage occurs in each connecting pipeline of the ventilator circuit or gas leakage occurs in an endotracheal intubation gas bag to reduce the pressure in the pipeline to be below the trigger sensitivity;
(4) vibration generated by heart beating: it is generally accompanied when the trigger sensitivity of the ventilator is set too low, and a change in the pressure in the lungs is caused during heart beating so as to trigger the ventilator to delivery gas.

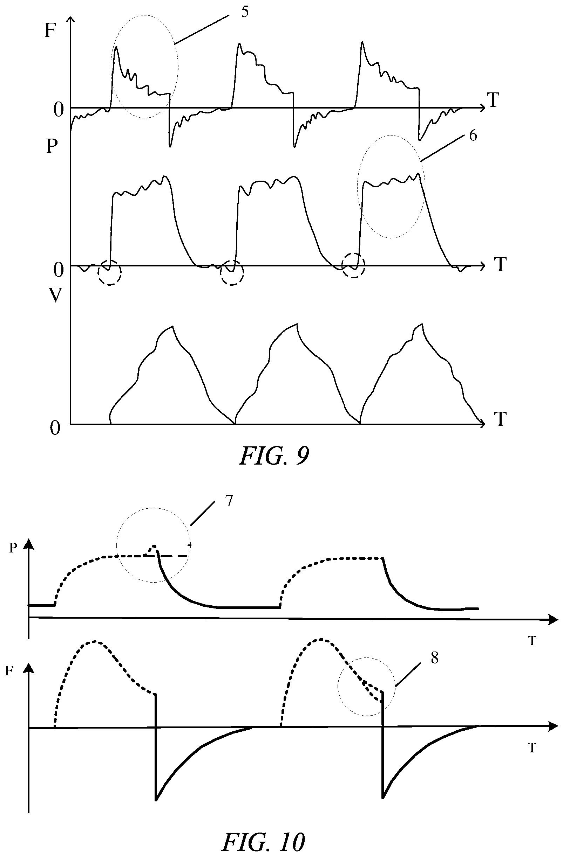

In the case of the false trigger described above, there are similar manifestations in the waveform of the airway pressure over time and the waveform of the airway flow over time. FIG. 9 is a schematic diagram of waveforms of the false trigger provided by an embodiment of the disclosure. Referring to FIG. 9, since the false trigger occurs, changes in the airway pressure and the airway flow may occur repeatedly during gas delivery of the ventilator, as shown by parts indicated by reference numbers 5-6 in FIG. 9.

In an embodiment, when the ventilator detects that at least one of the following situations has occurred by analyzing waveforms of the acquired ventilation parameters (the airway pressure and the airway flow) over time, it may be determined that an abnormal event of false trigger has occurred on the ventilator, that is, it may be determined that the change in the ventilation parameters meets corresponding parameter change conditions:
the airway pressure reaches the pressure trigger sensitivity of the ventilator, and the change in the magnitude of the airway pressure occurs repeatedly during the gas delivery of the ventilator; and
the airway flow reaches the flow trigger sensitivity of the ventilator, and the change in the magnitude of the airway flow occurs repeatedly during the gas delivery of the ventilator.

In an embodiment, a pressure-time waveform graph and a flow-time waveform graph corresponding to the false trigger event may be stored in the ventilator, a corresponding waveform graph may be obtained by monitoring the airway pressure and/or airway flow during the mechanical ventilation by the ventilator, and the obtained waveform graph is matched with the stored corresponding waveform graph of false trigger. For example, the obtained pressure-time waveform graph may be matched with the stored pressure-time waveform graph (within one respiratory cycle) corresponding to the false trigger, and when the similarity obtained by matching reaches a similarity threshold (e.g., 0.9), it may be determined that an abnormal event of inspiratory false trigger has occurred on the ventilator, and thus abnormality prompt information indicating that false trigger has occurred on the ventilator is sent.

In an embodiment, the ventilator may send abnormality prompt information, by means of a UI and/or in the form of sound, indicating that the false trigger has occurred on the ventilator when determining that the abnormal event of false trigger has occurred, so that the user adjusts the trigger sensitivity or repair the line to better realize the patient-ventilator synchronization.

With regard to abnormal inspiration-to-expiration switching, the inspiration-to-expiration switching may be controlled by the medullary respiratory center and may be an involuntary motion, and the abnormal inspiration-to-expiration switching will be caused when the setting of the inspiration-to-expiration switching during the mechanical ventilation does not meet the demand of the patient. The situation that the ventilator detects either early switching or delayed switching may prompt that the flow-based switching percentage of the ventilator is set improperly.

With regard to delayed cycling in the abnormal inspiration-to-expiration switching, the switching flow may be set too low for a patient suffering from tachypnea, so that the inspiratory time is prolonged, the patient may need to do additional exhalation work, and end inspiratory pressure overshoot may be generated in the waveform, or reduction of the airway flow may be suddenly accelerated; FIG. 10 is a schematic diagram of waveforms of delayed cycling provided by an embodiment of the disclosure. Referring to FIG. 10, a part indicated by a reference number 7 in FIG. 10 may refer to that the patient begins to exhale at the end of inhalation, but the ventilator has not switched to the expiratory phase at this time, so that the airway pressure instantaneously increases to generate an end inspiratory pressure overshoot. A part indicated by a reference number 8 in FIG. 10 may refer to that the patient begins to exhale at the end of inhalation, but the ventilator has not switched to the expiratory phase at this time. The exhalation effort may be not sufficient to cause a significant change in pressure, but a sudden drop in the flow, that is, a sudden increase in the change rate of the flow over time, may be obviously detected.

When the ventilator detects that at least one of the following situations has occurred by analyzing waveforms of the acquired ventilation parameters (the airway pressure and the airway flow) over time, it may be determined that an abnormal event of delayed cycling has occurred on the ventilator, that is, it may be determined that the change in the ventilation parameters meets corresponding parameter change conditions:
the situation of the end inspiratory pressure overshoot occurs in the waveform of the airway pressure over time; and
in the waveform of the airway flow over time, the situation occurs that the change rate of the airway flow exceeds a preset change rate threshold before the inspiration-to-expiration switching.

In an embodiment, the ventilator may determine the occurrence of the delayed cycling when detecting that a rise appears in the airway pressure-waveform or an accelerated drop occurs in a ventilation flow at an inspiration-to-expiration transitional stage.

In an embodiment, a pressure-time waveform graph and a flow-time waveform graph corresponding to the delayed cycling event may be stored in the ventilator, a corresponding waveform graph may be obtained by monitoring the airway pressure and/or airway flow during the mechanical ventilation by the ventilator, and the obtained waveform graph may be matched with the stored corresponding waveform graph of delayed cycling. For example, the obtained pressure-time waveform graph may be matched with the stored pressure-time waveform graph (within one respiratory cycle) corresponding to the delayed cycling, and when the similarity obtained by matching reaches a similarity threshold (e.g., 0.9), it may be determined that an abnormal event of inspiratory delayed cycling has occurred on the ventilator, and thus abnormality prompt information indicating that delayed cycling has occurred on the ventilator is sent.

In an embodiment, the ventilator may send abnormality prompt information, by means of a UI and/or in the form of sound, indicating that the delayed cycling has occurred on the ventilator when determining that the abnormal event of delayed cycling (delayed cycling) has occurred, so that the user adjusts the trigger sensitivity to better realize the patient-ventilator synchronization.

A preset trigger sensitivity adjusting strategy is adopted to adjust the trigger sensitivity of the ventilator when the ventilator determines that the abnormal event of delayed cycling has occurred, thats is, the trigger sensitivity of the ventilator is periodically increased (i.e., in each subsequent respiratory cycle, the trigger sensitivity is increased to 110% of that in a previous cycle each time) until no delayed cycling event occurs.

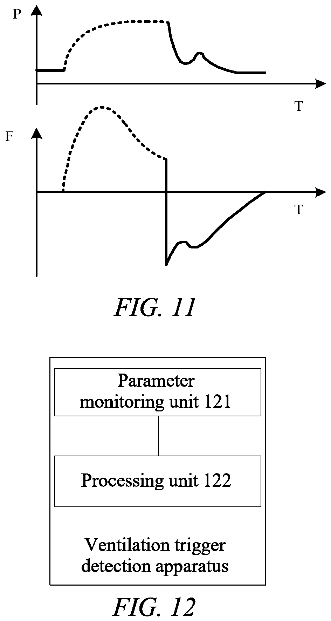

With regard to premature cycling in the abnormal inspiration-to-expiration switching, since the exhalatory trigger sensitivity (a percentage of the peak flow) of the ventilator may be set too high, the ventilator stops delivering the gas and may be switched for expiration when the patient still performs the inhalation action, causing insufficient inspiration for the patient and the patient-ventilator incoordination. It may be manifested by the flow-time waveform that a descending inspiratory branch may decrease to zero in advance and become an expiratory one, while the expiratory phase at an initial stage has the trend of rising again. If the patient's inhalation effort is strong, repeated trigger of the ventilator may be caused. The early switching may lead to the reduction of the tidal volume and polypnea of the patient, shortening the inspiratory time and increasing the respiration work of the patient. FIG. 11 is a schematic diagram of waveforms of premature cycling provided by an embodiment of the disclosure.

When the ventilator detects that at least one of the following situations has occurred by analyzing waveforms of the acquired ventilation parameters (the airway pressure and the airway flow) over time, it may be determined that an abnormal event of premature cycling has occurred on the ventilator, that is, it may be determined that the change in the ventilation parameters meets corresponding parameter change conditions:
the situation that the airway pressure rises and then drops after the inspiration-to-expiration switching occurs in the waveform of the airway pressure over time; and
in the waveform of the airway flow over time, the situation occurs that the airway flow increases and then decreases after the inspiration-to-expiration switching.

In an embodiment, the ventilator may determine the occurrence of the premature cycling when detecting the situation that a non-monotonic drop appears in the airway pressure-time waveform or a non-monotonic rise appears in the airway flow-time waveform at the inspiration-to-expiration transitional stage.

A pressure-time waveform graph and a flow-time waveform graph corresponding to the premature cycling event may be stored in the ventilator, a corresponding waveform graph may be obtained by monitoring the airway pressure and/or airway flow during the mechanical ventilation by the ventilator, and the obtained waveform graph may be matched with the stored corresponding waveform graph of premature cycling. For example, the obtained pressure-time waveform graph may be matched with the stored pressure-time waveform graph (within one respiratory cycle) corresponding to the premature cycling, and when the similarity obtained by matching reaches a similarity threshold (e.g., 0.9), it may be determined that an abnormal event of inspiratory premature cycling has occurred on the ventilator, and thus abnormality prompt information indicating that premature cycling has occurred on the ventilator is sent.

In an embodiment, the ventilator may send abnormality prompt information, by means of a UI and/or in the form of sound, indicating that the premature cycling has occurred on the ventilator when determining that the abnormal event of premature cycling has occurred, so that the user adjusts the trigger sensitivity to better realize the patient-ventilator synchronization.

A preset trigger sensitivity adjusting strategy is adopted to adjust the trigger sensitivity of the ventilator when the ventilator determines that the abnormal event of premature cycling has occurred, for example, the trigger sensitivity of the ventilator is periodically reduced (i.e., in each subsequent respiratory cycle, the trigger sensitivity is reduced to 90% of that in a previous cycle each time) until no premature cycling event occurs.

Continuing to describe the ventilation trigger detection apparatus provided by the embodiment of the disclosure, as an example of hardware implementation or software implementation of the ventilator, the ventilation trigger detection apparatus may be provided as a series of modules having a coupling relationship at a signal/information/data level, which will be described below with reference to FIG. 12. Referring to FIG. 12, FIG. 12 is an optional schematic diagram of constitution of the ventilation trigger detection apparatus provided by the embodiment of the disclosure, showing a series of units included in implementing the ventilation trigger detection apparatus, but the structure of the units of the ventilation trigger detection apparatus is not only limited to that shown in FIG. 12, for example, the units therein may be further separated or combined according to different functions to be achieved. Referring to FIG. 12, the ventilation trigger detection apparatus includes:
a parameter monitoring unit 121 may be configured to monitor a ventilation parameter during mechanical ventilation for a user, the ventilation parameter may include at least one of an airway pressure and an airway flow; and
a processing unit 122 may be configured to determine patient-ventilator synchrony during the ventilation according to a change in the ventilation parameter.

In an embodiment, the processing unit may be further configured to analyze a change trend of the obtained ventilation parameter;
and to determine whether patient-ventilator out-of-synchronization occurs during the ventilation according to the change trend of the ventilation parameter.

**In** an embodiment, the processing unit may be further configured to determine the type of the patient-ventilator out-of-synchronization according to the change trend of the ventilation parameter.

**In** an embodiment, the type of the patient-ventilator out-of-synchronization includes one or more of ineffective effort, double trigger, false inspiratory trigger, delayed cycling and premature cycling.

**In** an embodiment, the processing unit may be further configured to determine the occurrence of the ineffective effort when detecting that a valley appears in an airway pressure-time waveform and/or an accelerated rise appears in a ventilation flow-time waveform at an expiratory stage, and inspiratory trigger of a ventilator is not enabled.

In an embodiment, the processing unit may be further configured to determine the occurrence of the double triggerdouble trigger when detecting that two inspiratory pressure waveforms appear in the airway pressure-time waveform at an inspiratory stage and/or a short-time expiratory cycle appears between two inspiratory cycles in the airway flow-time waveform.

In an embodiment, the processing unit may be further configured to determine the occurrence of the delayed cycling when detecting that a rise appears in the airway pressure-time waveform or an accelerated drop occurs in a ventilation flow-time waveform at an inspiration-to-expiration transitional stage.

In an embodiment, the processing unit may be further configured to determine the occurrence of the premature cycling when detecting that a non-monotonic drop appears in the airway pressure-time waveform or a non-monotonic rise appears in the airway flow-time waveform at the inspiration-to-expiration transitional stage.

In an embodiment, the processing unit may be further configured to adjust ventilation trigger setting of the ventilation device or output prompt information about the patient-ventilator out-of-synchronization according to the determined type of the patient-ventilator out-of-synchronization.

An embodiment of the disclosure may further provide a readable storage medium. The storage medium may include: a mobile storage device, a random access memory (RAM), a read-only memory (ROM), a magnetic disk or an optical disk and other media which are capable of storing program codes. The readable storage medium stores executable instructions.

The executable instructions may be configured to implement the ventilation trigger detection method of the example of the disclosure when being executed by the processor.

It should be noted that: the above description relating to the ventilation trigger detection apparatus is similar to the description of the above method and the same as the description of the beneficial effects of the method, which will not be described in detail. Technical details not disclosed in the embodiments of the ventilation trigger detection apparatus according to the disclosure may be referred to the description of the examples of the method according to the disclosure.

All or some of the steps of the embodiments may be completed by a program that instructs related hardware. The program may be stored in a computer readable storage medium. When the program is executed, the steps including the above method examples are performed. The foregoing storage medium includes: a mobile storage device, a random access memory, a read-only memory, a magnetic disk or an optical disk and other media which are capable of storing program codes.

Alternatively, if implemented in the form of a software function module and sold or used as an independent product, the above integrated unit of the disclosure may also be stored in a computer readable storage medium. Based on such an understanding, the technical solutions in the embodiments of the disclosure essentially, or the part contributing to the related art may be implemented in a form of a software product. The computer software product is stored in a storage medium, including several instructions for instructing a computer device (which may be a personal computer, a server, or a network device) to perform all or some of the methods in the examples of the disclosure. The foregoing storage medium includes: a mobile storage device, a RAM, a ROM, a magnetic disk or an optical disk and other media which are capable of storing program codes.

The above descriptions are merely specific embodiments of the disclosure, but the scope of protection of the disclosure is not limited thereto. Changes or substitutions can be readily figured out by those skilled in the art within the technical scope of the disclosure. The invention is defined by the claims which follow.

## Claims

1. A computer program for ventilation trigger detection comprises instructions to cause a ventilation device to execute the steps of:
monitoring ventilation parameters during a mechanical ventilation for a user, the ventilation parameters being an airway pressure and an airway flow velocity; and
determining a patient-ventilator synchrony during the mechanical ventilation according to a change in the ventilation parameters by determining whether a change in the ventilation parameters meets a parameter change condition,
acquiring waveforms of each of the ventilation parameters over time during the mechanical ventilation;
carrying out similarity matching on the acquired waveforms of the ventilation parameters and stored waveforms of each of the corresponding ventilation parameters to obtain a similarity between the acquired waveforms of the ventilation parameters and the stored waveforms of the corresponding ventilation parameters, and
determining that the change in the ventilation parameters meets the parameter change condition when the similarity obtained by the matching reaches a waveform similarity threshold; and
wherein the acquired waveforms of the ventilation parameters and the stored waveforms of the corresponding ventilation parameters comprise an airway pressure-time waveform and an airway flow-time waveform corresponding to one or more of:
an ineffective effort event;
a double trigger event;
a false trigger event;
a delayed cycling event;
and a premature cycling event, and further
determining a patient-ventilator out-of-synchronization, when the change in the ventilation parameter meets the parameter change condition, and:
adjusting a ventilation trigger setting of the ventilation device after a type of the patient-ventilator out-of-synchronization is determined according to a change trend of the ventilation parameters, or
outputting a prompt information about the patient-ventilator out-of-synchronization according to the determined type of the patient-ventilator out-of-synchronization after a type of the patient-ventilator out-of-synchronization is determined according to a change trend of the ventilation parameters; and
periodically reducing an inspiratory trigger sensitivity of the ventilation device in each subsequent respiratory cycle when an ineffective effort occurs until no ineffective effort event occurs;
periodically reducing an inspiratory trigger sensitivity of the ventilation device in each subsequent respiratory cycle when an inspiratory double trigger occurs until no double trigger event occurs;
periodically increasing an inspiratory trigger sensitivity of the ventilation device in each subsequent respiratory cycle when a delayed expiratory cycling occurs until no delayed cycling event occurs; and
periodically reducing an inspiratory trigger sensitivity of the ventilation device in each subsequent respiratory cycle when a premature expiratory cycling occurs until no premature cycling event occurs.

2. A ventilation trigger detection apparatus (100) applied to a ventilation device, **characterized in that** the apparatus comprising:
a parameter monitoring unit (121) configured to monitor ventilation parameters during mechanical ventilation for a user, the ventilation parameters being an airway pressure and an airway flow velocity; and
a processing unit (122) configured to determine a patient-ventilator synchrony during the mechanical ventilation according to a change in the ventilation parameters by determining whether a change in the ventilation parameters meets a parameter change condition, wherein the processing unit (122) is further configured to:
acquire a waveform of each of the ventilation parameters over time during the mechanical ventilation;
carry out similarity matching on the acquired waveforms of the ventilation parameters and stored waveforms of each of the corresponding ventilation parameters to obtain a similarity between the acquired waveforms of the ventilation parameters and the stored waveforms of the corresponding ventilation parameters, and
determine that the change in the ventilation parameters meets the parameter change condition when the similarity obtained by the matching reaches a waveform similarity threshold; and
wherein the acquired waveforms of the ventilation parameters and the stored waveforms of the corresponding ventilation parameters comprise an airway pressure-time waveform and an airway flow-time waveform corresponding to one or more of:
an ineffective effort event;
a double trigger event;
a false trigger event;
a delayed cycling event; and
a premature cycling event; and
the processing unit (122) is further configured
to determine a patient-ventilator out-of-synchronization, when the change in the ventilation parameter meets the parameter change condition, and the processing unit (122) is further configured to
adjust a ventilation trigger setting of the ventilation device after a type of the patient-ventilator out-of-synchronization is determined according to a change trend of the ventilation parameters, or
output a prompt information about the patient-ventilator out-of-synchronization according to the determined type of the patient-ventilator out-of-synchronization after a type of the patient-ventilator out-of-synchronization is determined according to a change trend of the ventilation parameters; and
the processing unit (122) is further configured to
periodically reduce an inspiratory trigger sensitivity of the ventilation device in each subsequent respiratory cycle when an ineffective effort occurs until no ineffective effort event occurs;
periodically reducing an inspiratory trigger sensitivity of the ventilation device in each subsequent respiratory cycle when an inspiratory double trigger occurs until no double trigger event occurs;
periodically increase an inspiratory trigger sensitivity of the ventilation device in each subsequent respiratory cycle when a delayed expiratory cycling occurs until no delayed cycling event occurs; and
periodically reduce an inspiratory trigger sensitivity of the ventilation device in each subsequent respiratory cycle when a premature expiratory cycling occurs until no premature cycling event occurs.

3. A ventilation device, comprising a ventilation trigger detection apparatus (100), a gas source (160), an inspiratory branch (170), an expiratory branch (180) and a respiration line (190), wherein
the gas source (160) supplies a gas during a mechanical ventilation;
the inspiratory branch (170) is connected to the gas source (160) to provide an inspiration path during the mechanical ventilation;
the expiratory branch (180) provides an expiration path during the mechanical ventilation;
the respiration line (190) is connected to the inspiratory branch (170) and the expiratory branch (180) respectively, and used for delivering the gas to a user or exhausting the gas from a user during the mechanical ventilation; and
the ventilation trigger detection apparatus (100) is connected to the inspiratory branch (170) and the expiratory branch (180) respectively, and comprises:
a parameter monitoring unit (121) configured to monitor ventilation parameters during the mechanical ventilation for a user, the ventilation parameters being an airway pressure and an airway flow velocity; and
a processing unit (122) configured to determine a patient-ventilator synchrony during the mechanical ventilation according to a change in the ventilation parameters by determining whether a change in the ventilation parameters meets a parameter change condition; **wherein** the processing unit (122) is further configured to:
acquire waveforms of each of the ventilation parameters over time during the mechanical ventilation;
carry out similarity matching on the acquired waveforms of the ventilation parameters and stored waveforms of each of the corresponding ventilation parameters to obtain a similarity between the acquired waveforms of the ventilation parameters and the stored waveforms of the corresponding ventilation parameters, and
determine that the change in the ventilation parameters meets the parameter change condition when the similarity obtained by the matching reaches a waveform similarity threshold; and
wherein the acquired waveforms of the ventilation parameters and the stored waveforms of the corresponding ventilation parameters comprise an airway pressure-time waveform and an airway flow-time waveform corresponding to one or more of:
an ineffective effort event;
a double trigger event;
a false trigger event;
a delayed cycling event; and
a premature cycling event, and
the processing unit (122) is further configured
to determine a patient-ventilator out-of-synchronization, when the change in the ventilation parameter meets the parameter change condition, and the processing unit (122) is further configured to
adjust a ventilation trigger setting of the ventilation device after a type of the patient-ventilator out-of-synchronization is determined according to a change trend of the ventilation parameters, or
output a prompt information about the patient-ventilator out-of-synchronization according to the determined type of the patient-ventilator out-of-synchronization after a type of the patient-ventilator out-of-synchronization is determined according to a change trend of the ventilation parameters; and
the processing unit (122) is further configured to
periodically reduce an inspiratory trigger sensitivity of the ventilation device in each subsequent respiratory cycle when an ineffective effort occurs until no ineffective effort event occurs;
periodically reducing an inspiratory trigger sensitivity of the ventilation device in each subsequent respiratory when an inspiratory double trigger occurs until no double trigger event occurs;
periodically increase an inspiratory trigger sensitivity of the ventilation device in each subsequent respiratory cycle when a delayed expiratory cycling occurs until no delayed cycling event occurs; and
periodically reduce an inspiratory trigger sensitivity of the ventilation device in each subsequent respiratory cycle when a premature expiratory cycling occurs until no premature cycling event occurs.

## Patentansprüche

1. Ein Computerprogramm zur Erkennung von Beatmungstriggern mit Anweisungen, die ein Beatmungsgerät dazu veranlassen, die folgenden Schritte auszuführen:
Überwachen der Beatmungsparameter während einer mechanischen Beatmung eines Patienten, wobei die Beatmungsparameter einem Atemwegsdruck und einer Atemwegsströmungsgeschwindigkeit entsprechen; und
Bestimmen der Synchronität von Patient und Beatmungsgerät während der mechanischen Beatmung anhand einer Änderung der Beatmungsparameter, indem festgestellt wird, ob eine Änderung der Beatmungsparameter eine Bedingung für eine Parameteränderung erfüllt;
Erfassen der Wellenformen jedes Beatmungsparameters im Laufe der Zeit während der mechanischen Beatmung;
Ausführen eines Ähnlichkeitsvergleichs der erfassten Wellenformen der Beatmungsparameter und gespeicherten Wellenformen der entsprechenden Beatmungsparameter, um eine Ähnlichkeit zwischen den erfassten Wellenformen der Beatmungsparameter und den gespeicherten Wellenformen der entsprechenden Beatmungsparameter zu ermitteln;
Feststellen, dass die Änderung der Beatmungsparameter die Bedingung für die Parameteränderung erfüllt, wenn die durch den Abgleich erzielte Ähnlichkeit einen Schwellenwert der Wellenformähnlichkeit erreicht;
wobei die erfassten Wellenformen der Beatmungsparameter und die gespeicherten Wellenformen der entsprechenden Beatmungsparameter eine Atemwegsdruck-Zeit-Wellenform und eine Atemwegsfluss-Zeit-Wellenform umfassen, die einem oder mehreren der folgenden Ereignisse entsprechen, wie:
einer erfolgslosen Anstrengung;
einem Doppeltrigger;
einem Fehltrigger;
einem verzögerten Zyklus;
und einem vorzeitigen Zyklus; und ferner:
Bestimmen einer Fehlsynchronisation zwischen Patient und Beatmungsgerät, wenn die Änderung des Beatmungsparameters die Bedingung der Parameteränderung erfüllt:
Anpassen einer Beatmungstrigger-Einstellung des Beatmungsgeräts, nachdem eine Art der Fehlsynchronisation zwischen Patient und Beatmungsgerät gemäß einem Änderungstrend der Beatmungsparameter bestimmt wurde, oder
Ausgabe einer Hinweisinformation über die Fehlsynchronisation zwischen Patient und Beatmungsgerät gemäß der bestimmten Art einer Fehlsynchronisation zwischen Patient und Beatmungsgerät, nachdem eine Art der Fehlsynchronisation gemäß einem Änderungstrend der Beatmungsparameter bestimmt wurde; und
regelmäßige Reduzierung der Empfindlichkeit des Einatmungstriggers des Beatmungsgeräts in jedem nachfolgenden Atemzyklus, wenn eine erfolglose Anstrengung auftritt, bis keine erfolglose Anstrengung mehr auftritt;
regelmäßige Reduzierung der Empfindlichkeit des Einatmungstriggers des Beatmungsgeräts in jedem nachfolgenden Atemzyklus, wenn ein Einatmungsdoppeltrigger auftritt, bis kein Doppeltrigger-Ereignis mehr auftritt;
regelmäßiges Erhöhen der Empfindlichkeit des Einatmungstriggers des Beatmungsgeräts in jedem nachfolgenden Atemzyklus, wenn ein verzögerter Ausatmungszyklus auftritt, bis kein weiteres verzögertes Zyklusereignis mehr auftritt;
regelmäßige Reduzierung der Empfindlichkeit des Einatmungstriggers des Beatmungsgeräts in jedem nachfolgenden Atemzyklus, wenn ein vorzeitiger Ausatmungs-Zyklus auftritt, bis kein weiterer vorzeitiger Zyklus mehr auftritt.

2. Eine Vorrichtung zur Erkennung des Beatmungstriggers (100), die in einem Beatmungsgerät verwendet wird, mit der Eigenschaft, dass die Vorrichtung Folgendes umfasst:
eine Parameterüberwachungseinheit (121), die so konfiguriert ist, dass sie während der mechanischen Beatmung eines Patienten Beatmungsparameter überwacht, wobei die Beatmungsparameter dem Atemwegsdruck und der Atemwegsdurchflussgeschwindigkeit entsprechen; und
eine Verarbeitungseinheit (122), die so konfiguriert ist, dass sie die Synchronität zwischen Patient und Beatmungsgerät während der mechanischen Beatmung gemäß einer Änderung der Beatmungsparameter bestimmt, indem festgestellt wird, ob eine Änderung der Beatmungsparameter eine Bedingung der Parameteränderung erfüllt; wobei die Verarbeitungseinheit (122) ferner so konfiguriert ist, dass sie:
eine Wellenform jedes Beatmungsparameters im Laufe der Zeit während der mechanischen Beatmung erfasst;
einen Ähnlichkeitsvergleich zwischen den erfassten Wellenformen der Beatmungsparameter und den gespeicherten Wellenformen der entsprechenden Beatmungsparameter ausführt, um eine Ähnlichkeit zwischen den erfassten Wellenformen der Beatmungsparameter und den gespeicherten Wellenformen der entsprechenden Beatmungsparameter zu bestimmen, und
feststellt, dass die Änderung der Beatmungsparameter die Bedingung der Parameteränderung erfüllt, wenn die durch den Abgleich erzielte Ähnlichkeit einen Schwellenwert der Wellenformähnlichkeit erreicht;
wobei die erfassten Wellenformen der Beatmungsparameter und die gespeicherten Wellenformen der entsprechenden Beatmungsparameter eine Atemwegsdruck-Zeit-Wellenform und eine Atemwegsfluss-Zeit-Wellenform umfassen, die einem oder mehreren der folgenden Ereignisse entsprechen, wie:
einer erfolgslosen Anstrengung;
einem Doppeltrigger;
einem Fehltrigger;
einem verzögerten Zyklus;
und einem vorzeitigen Zyklus; und
die Verarbeitungseinheit (122) ferner so konfiguriert ist, dass sie:
eine Fehlsynchronisation zwischen Patient und Beatmungsgerät bestimmt, wenn die Änderung des Beatmungsparameters die Bedingung der Parameteränderung erfüllt,
die Beatmungstrigger-Einstellung des Beatmungsgeräts anpasst, nachdem eine Art der Fehlsynchronisation zwischen Patient und Beatmungsgerät gemäß einem Änderungstrend der Beatmungsparameter bestimmt wurde, oder
eine Hinweisinformation über die Fehlsynchronisation zwischen Patient und Beatmungsgerät gemäß der bestimmten Art der Fehlsynchronisation ausgibt, nachdem eine Art der Fehlsynchronisation gemäß einem Änderungstrend der Beatmungsparameter bestimmt wurde;
die Empfindlichkeit des Einatmungstriggers des Beatmungsgeräts in jedem nachfolgenden Atemzyklus regelmäßig reduziert, wenn eine erfolglose Anstrengung auftritt, bis keine erfolglose Anstrengung mehr auftritt;
die Empfindlichkeit des Einatmungstriggers des Beatmungsgeräts in jedem nachfolgenden Atemzyklus regelmäßig reduziert, wenn ein Einatmungsdoppeltrigger auftritt, bis kein Doppeltrigger-Ereignis mehr auftritt
die Empfindlichkeit des Einatmungstriggers des Beatmungsgeräts in jedem nachfolgenden Atemzyklus regelmäßig erhöht, wenn ein verzögerter Ausatmungszyklus auftritt, bis kein weiteres verzögertes Zyklusereignis mehr auftrit;
die Empfindlichkeit des Einatmungstriggers des Beatmungsgeräts in jedem nachfolgenden Atemzyklus regelmäßig reduziert, wenn ein vorzeitiger Ausatmungs-Zyklus auftritt, bis kein weiterer vorzeitiger Zyklus mehr auftritt.

3. Ein Beatmungsgerät mit einer Vorrichtung zur Erkennung des Beatmungstriggers (100), einer Gasquelle (160), einem Inspirationsschenkel (170), einem Exspirationsschenkel (180), und einer Atemleitung (190), wobei:
die Gasquelle (160) während der mechanischen Beatmung Gas liefert;
der Inspirationsschenkel (170) mit der Gasquelle (160) verbunden ist, um während der mechanischen Beatmung einen Einatmungsweg zu schaffen;
der Exspirationsschenkel (180) während der mechanischen Beatmung einen Ausatmungsweg schafft;
die Atemleitung (190) sowohl mit dem Inspirationsschenkel (170) als auch mit dem Exspirationsschenkel (180) verbunden ist und verwendet wird, um einem Patienten während der mechanischen Beatmung Gas zuzuführen oder von ihm abzuleiten;
die Vorrichtung zur Erkennung des Beatmungstriggers (100) jeweils mit dem Inspirationsschenkel (170) und dem Exspirationsschenkel (180) verbunden ist und Folgendes umfasst:
eine Parameterüberwachungseinheit (121), die so konfiguriert ist, dass sie während der mechanischen Beatmung eines Patienten Beatmungsparameter überwacht, wobei die Beatmungsparameter dem Atemwegsdruck und der Atemwegsdurchflussgeschwindigkeit entsprechen;
eine Verarbeitungseinheit (122), die so konfiguriert ist, dass sie die Synchronität zwischen Patient und Beatmungsgerät während der mechanischen Beatmung gemäß einer Änderung der Beatmungsparameter bestimmt, indem festgestellt wird, ob eine Änderung der Beatmungsparameter eine Bedingung der Parameteränderung erfüllt;
wobei die Verarbeitungseinheit (122) ferner so konfiguriert ist, dass sie:
Wellenformen jedes Beatmungsparameters im Laufe der Zeit während der mechanischen Beatmung erfasst;
einen Ähnlichkeitsvergleich zwischen den erfassten Wellenformen der Beatmungsparameter und den gespeicherten Wellenformen der entsprechenden Beatmungsparameter ausführt, um eine Ähnlichkeit zwischen den erfassten Wellenformen der Beatmungsparameter und den gespeicherten Wellenformen der entsprechenden Beatmungsparameter zu bestimmen;
feststellt, dass die Änderung der Beatmungsparameter die Bedingung der Parameteränderung erfüllt, wenn die durch den Abgleich erzielte Ähnlichkeit einen Schwellenwert der Wellenformähnlichkeit erreicht;
wobei die erfassten Wellenformen der Beatmungsparameter und die gespeicherten Wellenformen der entsprechenden Beatmungsparameter eine Atemwegsdruck-Zeit-Wellenform und
eine Atemwegsfluss-Zeit-Wellenform umfassen, die einem oder mehreren der folgenden Ereignisse entsprechen, wie:
einer erfolgslosen Anstrengung;
einem Doppeltrigger;
einem Fehltrigger;
einem verzögerten Zyklus;
und einem vorzeitigen Zyklus; und
die Verarbeitungseinheit (122) ferner so konfiguriert ist, dass sie:
eine Fehlsynchronisation zwischen Patient und Beatmungsgerät bestimmt, wenn die Änderung des Beatmungsparameters die Bedingung der Parameteränderung erfüllt,
die Beatmungstrigger-Einstellung des Beatmungsgeräts anpasst, nachdem eine Art der Fehlsynchronisation zwischen Patient und Beatmungsgerät gemäß einem Änderungstrend der Beatmungsparameter bestimmt wurde, oder
eine Hinweisinformation über die Fehlsynchronisation zwischen Patient und Beatmungsgerät gemäß der bestimmten Art der Fehlsynchronisation ausgibt, nachdem eine Art der Fehlsynchronisation gemäß einem Änderungstrend der Beatmungsparameter bestimmt wurde;
wobei die Verarbeitungseinheit (122) ferner so konfiguriert ist, dass sie:
die Empfindlichkeit des Einatmungstriggers des Beatmungsgeräts in jedem nachfolgenden Atemzyklus regelmäßig reduziert, wenn eine erfolglose Anstrengung auftritt, bis keine erfolglose Anstrengung mehr auftritt;
die Empfindlichkeit des Einatmungstriggers des Beatmungsgeräts in jedem nachfolgenden Atemzyklus regelmäßig reduziert, wenn ein Einatmungsdoppeltrigger auftritt, bis kein Doppeltrigger-Ereignis mehr auftritt
die Empfindlichkeit des Einatmungstriggers des Beatmungsgeräts in jedem nachfolgenden Atemzyklus regelmäßig erhöht, wenn ein verzögerter Ausatmungszyklus auftritt, bis kein weiteres verzögertes Zyklusereignis mehr auftrit;
die Empfindlichkeit des Einatmungstriggers des Beatmungsgeräts in jedem nachfolgenden Atemzyklus regelmäßig reduziert, wenn ein vorzeitiger Ausatmungs-Zyklus auftritt, bis kein weiterer vorzeitiger Zyklus mehr auftritt.

## Revendications

1. Programme informatique pour la détection de déclenchement de ventilation, comprenant des instructions pour amener un dispositif de ventilation à exécuter les étapes consistant à :
surveiller des paramètres de ventilation durant une ventilation mécanique pour un utilisateur, les paramètres de ventilation étant une pression de voies respiratoires et une vitesse d'écoulement de voies respiratoires ; et
déterminer une synchronisation patent-ventilateur durant la ventilation mécanique selon une variation des paramètres de ventilation en déterminant si une variation des paramètres de ventilation répond à une condition de variation de paramètre,
acquérir des formes d'onde de chacun des paramètres de ventilation au fil du temps durant la ventilation mécanique ;
réaliser une mise en correspondance de similarité sur les formes d'onde acquises des paramètres de ventilation et des formes d'onde enregistrées de chacun des paramètres de ventilation correspondants pour obtenir une similarité entre les formes d'onde acquises des paramètres de ventilation et les formes d'onde enregistrées des paramètres de ventilation correspondants, et
déterminer que la variation des paramètres de ventilation répond à la condition de variation de paramètre lorsque la similarité obtenue par la mise en correspondance atteint un seuil de similarité de forme d'onde ; et
où les formes d'onde acquises des paramètres de ventilation et les formes d'onde enregistrées des paramètres de ventilation correspondants comprennent une forme d'onde de pression de voies respiratoires en fonction du temps et une forme d'onde de débit de voies respiratoires en fonction du temps correspondant à un ou plusieurs événements parmi :
un événement d'effort inefficace ;
un événement de double déclenchement ;
un événement de faux déclenchement ;
un événement de cycle retardé ;
et un événement de cycle prématuré, et en outre
déterminer une désynchronisation patient-ventilateur, lorsque la variation du paramètre de ventilation répond à la condition de variation de paramètre, et :
ajuster un réglage de déclenchement de ventilation du dispositif de ventilation après qu'un type de la désynchronisation patient-ventilateur est déterminé selon une tendance de variation des paramètres de ventilation, ou
fournir une information d'invite concernant la désynchronisation patient-ventilateur selon le type déterminé de la désynchronisation patient-ventilateur après qu'un type de la désynchronisation patient-ventilateur est déterminé selon une tendance de variation des paramètres de ventilation ; et
réduire périodiquement une sensibilité de déclenchement inspiratoire du dispositif de ventilation dans chaque cycle respiratoire ultérieur lorsqu'un effort inefficace survient jusqu'à ce qu'aucun événement d'effort inefficace ne survienne ;
réduire périodiquement une sensibilité de déclenchement inspiratoire du dispositif de ventilation dans chaque cycle respiratoire ultérieur lorsqu'un double déclenchement inspiratoire survient jusqu'à ce qu'aucun événement de double déclenchement ne survienne ;
augmenter périodiquement une sensibilité de déclenchement inspiratoire du dispositif de ventilation dans chaque cycle respiratoire ultérieur lorsqu'un cycle expiratoire retardé survient jusqu'à ce qu'aucun événement de cycle retardé ne survienne ; et
réduire périodiquement une sensibilité de déclenchement inspiratoire du dispositif de ventilation dans chaque cycle respiratoire ultérieur lorsqu'un cycle expiratoire prématuré survient jusqu'à ce qu'aucun événement de cycle prématuré ne survienne.

2. Appareil de détection de déclenchement de ventilation (100) appliqué à un dispositif de ventilation, **caractérisé en ce que** l'appareil comprend :
une unité de surveillance de paramètre (121) configurée pour surveiller des paramètres de ventilation durant une ventilation mécanique pour un utilisateur, les paramètres de ventilation étant une pression de voies respiratoires et une vitesse d'écoulement de voies respiratoires ; et
une unité de traitement (122) configurée pour déterminer une synchronisation patient-ventilateur durant la ventilation mécanique selon une variation des paramètres de ventilation en déterminant si une variation des paramètres de ventilation répond à une condition de variation de paramètre, où l'unité de traitement (122) est en outre configurée pour :
acquérir une forme d'onde de chacun des paramètres de ventilation au fil du temps durant la ventilation mécanique ;
réaliser une mise en correspondance de similarité sur les formes d'onde acquises des paramètres de ventilation et des formes d'onde enregistrées de chacun des paramètres de ventilation correspondants pour obtenir une similarité entre les formes d'onde acquises des paramètres de ventilation et les formes d'onde enregistrées des paramètres de ventilation correspondants, et
déterminer que la variation des paramètres de ventilation répond à la condition de variation de paramètre lorsque la similarité obtenue par la mise en correspondance atteint un seuil de similarité de forme d'onde ; et
où les formes d'onde acquises des paramètres de ventilation et les formes d'onde enregistrées des paramètres de ventilation correspondants comprennent une forme d'onde de pression de voies respiratoires en fonction du temps et une forme d'onde de débit de voies respiratoires en fonction du temps correspondant à un ou plusieurs événements parmi :
un événement d'effort inefficace ;
un événement de double déclenchement ;
un événement de faux déclenchement ;
un événement de cycle retardé ; et
un événement de cycle prématuré ; et
l'unité de traitement (122) est en outre configurée
pour déterminer une désynchronisation patient-ventilateur, lorsque la variation du paramètre de ventilation répond à la condition de variation de paramètre, et l'unité de traitement (122) est en outre configurée pour
ajuster un réglage de déclenchement de ventilation du dispositif de ventilation après qu'un type de la désynchronisation patient-ventilateur est déterminé selon une tendance de variation des paramètres de ventilation, ou
fournir une information d'invite concernant la désynchronisation patient-ventilateur selon le type déterminé de la désynchronisation patient-ventilateur après qu'un type de la désynchronisation patient-ventilateur est déterminé selon une tendance de variation des paramètres de ventilation ; et
l'unité de traitement (122) est en outre configurée pour
réduire périodiquement une sensibilité de déclenchement inspiratoire du dispositif de ventilation dans chaque cycle respiratoire ultérieur lorsqu'un effort inefficace survient jusqu'à ce qu'aucun événement d'effort inefficace ne survienne ;
réduire périodiquement une sensibilité de déclenchement inspiratoire du dispositif de ventilation dans chaque cycle respiratoire ultérieur lorsqu'un double déclenchement inspiratoire survient jusqu'à ce qu'aucun événement de double déclenchement ne survienne ;
augmenter périodiquement une sensibilité de déclenchement inspiratoire du dispositif de ventilation dans chaque cycle respiratoire ultérieur lorsqu'un cycle expiratoire retardé survient jusqu'à ce qu'aucun événement de cycle retardé ne survienne ; et
réduire périodiquement une sensibilité de déclenchement inspiratoire du dispositif de ventilation dans chaque cycle respiratoire ultérieur lorsqu'un cycle expiratoire prématuré survient jusqu'à ce qu'aucun événement de cycle prématuré ne survienne.

3. Dispositif de ventilation, comprenant un appareil de détection de déclenchement de ventilation (100), une source de gaz (160), une branche inspiratoire (170), une branche expiratoire (180) et une ligne de respiration (190), où
la source de gaz (160) fournit un gaz durant une ventilation mécanique ;
la branche inspiratoire (170) est reliée à la source de gaz (160) pour fournir une voie d'inspiration durant la ventilation mécanique ;
la branche expiratoire (180) fournit une voie d'expiration durant la ventilation mécanique ;
la ligne de respiration (190) est reliée à la branche inspiratoire (170) et à la branche expiratoire (180) respectivement, et utilisée pour délivrer le gaz à un utilisateur ou évacuer le gaz d'un utilisateur durant la ventilation mécanique ; et
l'appareil de détection de déclenchement de ventilation (100) est relié à la branche inspiratoire (170) et à la branche expiratoire (180) respectivement, et comprend :
une unité de surveillance de paramètre (121) configurée pour surveiller des paramètres de ventilation durant la ventilation mécanique pour un utilisateur, les paramètres de ventilation étant une pression de voies respiratoires et une vitesse d'écoulement de voies respiratoires ; et
une unité de traitement (122) configurée pour déterminer une synchronisation patient-ventilateur durant la ventilation mécanique selon une variation des paramètres de ventilation en déterminant si une variation des paramètres de ventilation répond à une condition de variation de paramètre, où l'unité de traitement (122) est en outre configurée pour :
acquérir des formes d'onde de chacun des paramètres de ventilation au fil du temps durant la ventilation mécanique ;
réaliser une mise en correspondance de similarité sur les formes d'onde acquises des paramètres de ventilation et des formes d'onde enregistrées de chacun des paramètres de ventilation correspondants pour obtenir une similarité entre les formes d'onde acquises des paramètres de ventilation et les formes d'onde enregistrées des paramètres de ventilation correspondants, et
déterminer que la variation des paramètres de ventilation répond à la condition de variation de paramètre lorsque la similarité obtenue par la mise en correspondance atteint un seuil de similarité de forme d'onde ; et
où les formes d'onde acquises des paramètres de ventilation et les formes d'onde enregistrées des paramètres de ventilation correspondants comprennent une forme d'onde de pression de voies respiratoires en fonction du temps et une forme d'onde de débit de voies respiratoires en fonction du temps correspondant à un ou plusieurs événements parmi :
un événement d'effort inefficace ;
un événement de double déclenchement ;
un événement de faux déclenchement ;
un événement de cycle retardé ; et
un événement de cycle prématuré, et
l'unité de traitement (122) est en outre configurée
pour déterminer une désynchronisation patient-ventilateur, lorsque la variation du paramètre de ventilation répond à la condition de variation de paramètre, et l'unité de traitement (122) est en outre configurée pour
ajuster un réglage de déclenchement de ventilation du dispositif de ventilation après qu'un type de la désynchronisation patient-ventilateur est déterminé selon une tendance de variation des paramètres de ventilation, ou
fournir une information d'invite concernant la désynchronisation patient-ventilateur selon le type déterminé de la désynchronisation patient-ventilateur après qu'un type de la désynchronisation patient-ventilateur est déterminé selon une tendance de variation des paramètres de ventilation ; et
l'unité de traitement (122) est en outre configurée pour
réduire périodiquement une sensibilité de déclenchement inspiratoire du dispositif de ventilation dans chaque cycle respiratoire ultérieur lorsqu'un effort inefficace survient jusqu'à ce qu'aucun événement d'effort inefficace ne survienne ;
réduire périodiquement une sensibilité de déclenchement inspiratoire du dispositif de ventilation dans chaque cycle respiratoire ultérieur lorsqu'un double déclenchement inspiratoire survient jusqu'à ce qu'aucun événement de double déclenchement ne survienne ;
augmenter périodiquement une sensibilité de déclenchement inspiratoire du dispositif de ventilation dans chaque cycle respiratoire ultérieur lorsqu'un cycle expiratoire retardé survient jusqu'à ce qu'aucun événement de cycle retardé ne survienne ; et
réduire périodiquement une sensibilité de déclenchement inspiratoire du dispositif de ventilation dans chaque cycle respiratoire ultérieur lorsqu'un cycle expiratoire prématuré survient jusqu'à ce qu'aucun événement de cycle prématuré ne survienne.
